# EUROPÄISCHE PATENTSCHRIFT

(11) **EP 0 581 925 B1**
(45) Veröffentlichungstag und Bekanntmachung des Hinweises auf die Patenterteilung: **08.11.1995**
(21) Anmeldenummer: 93903140.7
(22) Anmeldetag: 12.02.1993
(51) Int. Cl.: A61M 5/178, A61M 5/24

(54) **ANZEIGEEINRICHTUNG FÜR EIN INJEKTIONSGERÄT**
INJECTOR DISPLAY
DISPOSITIF D'AFFICHAGE POUR UN APPAREIL A INJECTIONS

(30) Priorität: 24.02.1992 CH 555/92
(43) Veröffentlichungstag der Anmeldung: 09.02.1994
(73) Patentinhaber: MEDIMPEX ETS., FL-9496 Balzers (LI)
(72) Erfinder: MICHEL, Peter, CH-3400 Burgdorf (CH)
(74) Vertreter: Lusuardi, Werther Giovanni, Dr.
(86) Internationale Anmeldenummer: CH9300038
(87) Internationale Veröffentlichungsnummer: WO9316743

(56) Entgegenhaltungen:
- WO-A-90/09202

## Beschreibung

Die Erfindung bezieht sich auf eine Anzeigeeinrichtung für ein medizinisches Injektionsgerät nach dem Oberbegriff des Patentanspruchs 1.

Injektionsgeräte (im folgenden kurz "Geräte" genannt) werden in der Medizin zum Injizieren von Injektionsflüssigkeiten beispielsweise in den menschlichen Körper verwendet. Die Anmeldung betrifft solche Geräte, mit denen Patienten sich selbst jederzeit Injektionen verabreichen können. Dabei spielt die Kleinheit des Gerätes, es soll in der Kleidung unterbringbar sein, die Möglichkeit, mehrere Injektionen mit einer im Gerät befindlichen Ampulle auszuführen und die jeweilige Injektionsdosis genau vorzuwählen, die Zuverlässigkeit und die leichte Handhabung eines solchem Gerätes eine für die Beschaffung entscheidende Rolle.

Solche Gerate sind bekannt. In der EP 0 293 958 B1 wird ein Gerät beschrieben, in das Ampullen, die einen Kolben aufweisen, eingesetzt werden. Der Kolben wird mittels eines von einem Elektromotor linear angetriebenen Stabes vorgeschoben und so die in der Ampulle befindliche Injektionsflüssigkeit bei der Injektion durch eine Nadel aus der Ampulle ausgetrieben. Die Verstellung des Stabes und damit des Kolbens, durch welche die Injektionsdosis bestimmt wird, ist über einen Sensor messbar. Die am Sensor gemessene elektrische Signalgrösse wird an einem Vergleicher mit einem vom Patienten vorher eingestellten elektrischen Sollwert für die Injektionsdosis, der an einer Anzeige darstellbar ist, verglichen. Ist die mit dem Sollwert eingestellte Dosis erreicht, wird der Elektromotor abgestellt. Beim Einstellen des Sollwertes wird jedesmal elektrisch geprüft, ob die eingestellte Dosis noch in der Ampulle vorhanden ist. Wenn nicht, wird der Elektromotor blockiert, damit die Injektion unmöglich gemacht und dies an der Anzeige gemeldet.

Dies Gerät kann zwar in der Kleidung eines Patienten mitgeführt werden, ist aber wegen des Elektromotors und des Getriebes noch ziemlich gross. Da vom Sensor zunächst ein elektrischer Analogwert geliefert wird, kann bei einer etwaigen Verstärkungsänderung im Gerät die durch den Sollwert gewünschte Verstellung des Stabes vom tatsächlich eingestellten Wert abweichen, so dass der Patient eine falsche Dosis injiziert. Er hat keine Möglichkeit, dies zu überprüfen. Auch fehlt eine Anzeige über den Ladezustand der Batterie, die beispielsweise vor einer notwendigen Injektion in einem Zustand sein kann, dass die Steuerung und die Anzeigen noch betätigbar sind, der Elektromotor aber nicht mehr anläuft und so die Injektion nicht mehr durchführbar ist. Der elektronische steuerungsaufwand in diesem Gerät, der noch eine Reihe hier nicht genannter Funktionen betrifft, ist gross.

Ein weiteres Gerät ist aus der WO 87/02895 bekannt. Es wird rein manuell betätigt und hat die Form und Abmessung eines Füllfederhalters mit Klipp, ist also leicht in der Kleidung eines Patienten mitführbar. Durch Drehen an einem Bedienknopf ist die Injektionsdosis an einem Stab einstellbar, dabei hat dieser Stab noch keine Berührung mit dem Kolben der auch hier verwendeten Ampulle, die mit Injektionsflüssigkeit gefüllt ist. Durch axiales Vorschieben des Bedienknopfes wird dann der Stab in Kontakt mit dem Kolben gebracht und durch diesen die eingestellte Dosis über eine Nadel ausgetrieben. Der Stab mit dem Bedienknopf springt dann wieder zurück, worauf durch Drehen am Bedienknopf die nächste Dosis einstellbar ist. Die Grösse der Dosis wird von der Umdrehungszahl des Bedienknopfes bestimmt, diese kann durch akustische Signale, die beispielsweise bei jeder Vierteldrehung auftreten, abgehört werden.

Die eingestellte Dosis ist bei diesem Gerät nur beim Einstellen akustisch, danach nicht mehr überprüfbar. Dies ist ein merklicher Nachteil, da Fehldosierungen nicht ausgeschlossen werden können.

Gegen die Unzulänglichkeiten beider Geräte will die Erfindung Abhilfe schaffen. Ihr liegt die Aufgabe zugrunde, für ein Injektionsgerät eine Anzeigeeinrichtung zu schaffen, durch die für den Patient nach Einstellen der gewünschten Dosis diese nachträglich fehlerfrei überprüfbar ist und er eine Information darüber erhält, wie viele Injektionsflüssigkeit noch für weitere Injektionen zur Verfügung steht.

Diese Aufgabe wird erfindungsgemäss durch die Merkmale des Anspruchs 1 gelöst. Die weiteren Ansprüche betreffen vorteilhafte Ausführungsformen.

Diese erfindungsgemässe Anzeigeeinrichtung wird an Hand eines Beispiels in der Zeichnung erklärt. Dabei zeigen die
- Fig. 1: einen Längsschnitt durch den bedienknopfseitigen Teil eines Injektionsgerätes mit einer erfindungsgemässen Anzeigeeinrichtung,
- Fig. 2: einen Querschnitt A - A durch das Gerät nach Fig. 1 und
- Fig. 3: eine Darstellung der Signalfolge der Anzeigeeinrichtung bei einer Drehbewegung eines Bedienknopfes.

In der Zeichnung werden gleichartige Teile mit gleichen Beizeichen gekennzeichnet.

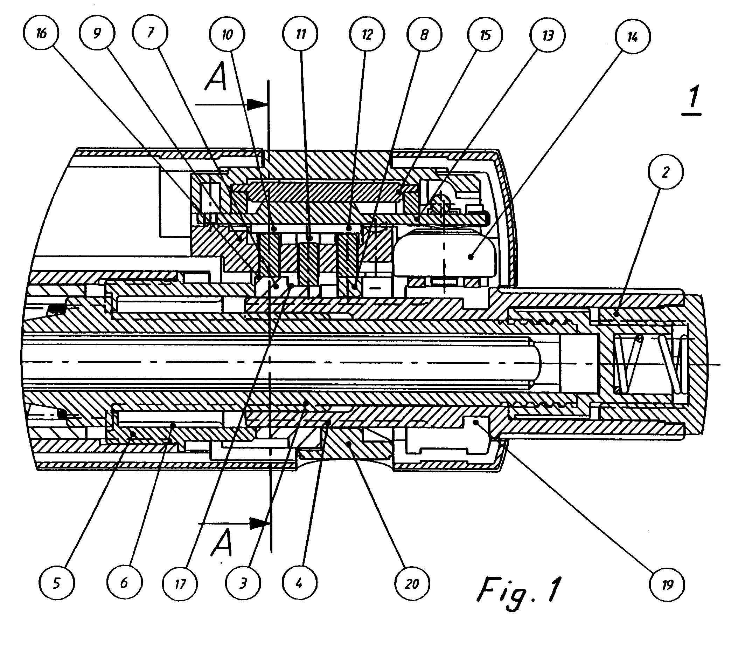

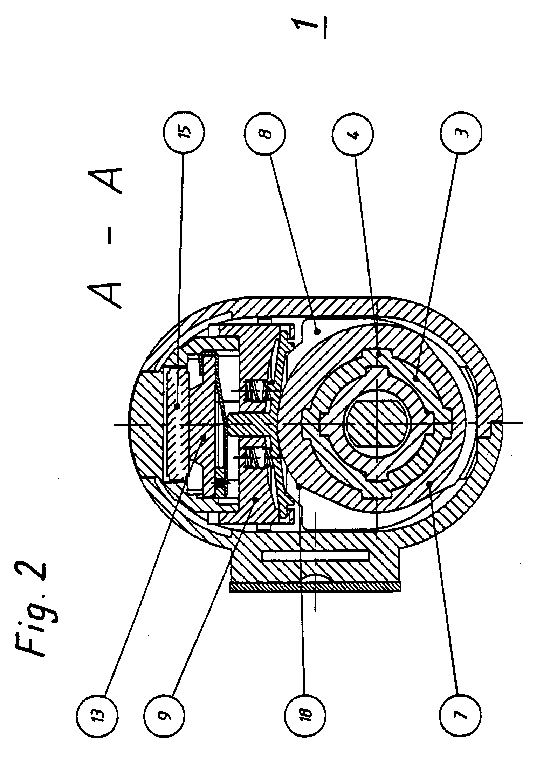

Eine erfindungsgemässe Anzeigeeinrichtung an einem in den Fig. 1 und 2 teilweise dargestellten Injektionsgerät 1 , das im übrigen beispielsweise ähnlich dem Gerät nach der WO 87/02895 gestaltet sein kann, weist einen axialen, einen zylindrischen Teil 3 besitzenden Bedienknopf 2 auf, an dem Drehbewegungen zur Einstellung der Grösse einer anschliessend zu injizierenden Injektionsdosis und axiale Bewegungen zum Durchführen der Injektion ausführbar sind. Die Drehbewegungen des Bedienknopfs 2 setzen sich aus zusammenhängenden Teildrehungen zusammen, von denen eine gegebene Zahl eine ganze Umdrehung ergibt. Beispielsweise können die Teildrehungen Vierteldrehungen sein.

Der Bedienknopf 2 kann nur axial bewegt werden, wenn er in einer Stellung einer abgeschlossenen Teildrehung steht. Der zylindrische Teil 3 des Bedienknopfes 2 besitzt nämlich eine der gegebenen Zahl der Teildrehungen entsprechende Zahl von in axialer Richtung verlaufender Längsnocken 4, die bei einer axialen Bewegung des Bedienknopfes 2 in ebenso vielen Längsnuten 6 einer Gehäusewand 5 eingreifen und dann eine Drehung verhindern. Die Endlage der axialen Bewegung, d.h. die Lage, in der die Längsnocken 4 ganz in die Längsnuten 6 eingeschoben sind, ist nicht gezeichnet, jedoch ihre Ruhelage, bei der eine Drehbewegung des Bedienknopfes 2 möglich ist, da dann kein Eingriff zwischen den Längsnocken 4 und den Längsnuten 6 besteht. Dazu wird der in die Endlage vorgeschobene Bedienknopf 2 durch elastische Mittel im nicht gezeichneten unteren Teil des Gerätes 1 in die Ruhelage zurückgeführt.

Auf dem Bedienknopf 2 sind Schaltmittel 7, 8 aufgelegt, die durch dessen Bewegung betätigbar sind. Sie greifen in ein Schalterstück 9 ein und betätigen ihrerseits dort Schalter 10, 11, 12. Das Schalterstück 9 ist elektrisch mit einer Leiterplatte 13, die durch eine Batterie 14 versorgt ist, und die Leiterplatte 13 elektrisch mit einer Anzeigeeinrichtung 15, beispielsweise einer LCD-Anzeigeeinrichtung, verbunden.

Diese Schaltmittel 7. 8 können Signale über die Drehbewegung, sie werden dann "Nockenringe" 7 genannt, oder Signale über die axiale Bewegung, sie werden dann "Schaltringe" 8 genannt, bilden. Vorzugsweise geht der zylindrische Teil 3 des Bedienknopfes 2 durch die Schaltmittel 7, 8 hindurch.

Die Nockenringe 7 drehen sich mit dem Bedienknopf 2, wobei sie beispielsweise durch die Längsnocken 4 auf dem zylindrischen Teil 3 des Bedienknopfes 2 mitgenommen werden. Es können aber auch andere Übertragungsarten für die Drehbewegung vorgesehen sein. Vorteilhafter Weise tragen sie gleichviele Nocken 18, die gleichmässig über ihren Umfang verteilt sind. Ein Nockenring 7 kann mehrere auf seinem äusseren Umfang nebeneinander liegende Nockenflächen 16, 17 tragen, deren Nocken 18 versetzt zueinander liegen. Trägt er, wie in der Zeichnung, eine erste Nockenfläche 16 und eine zweite Nockenfläche 17, so betätigt die erste Nockenfläche 16 einen ersten Schalter 10 und die zweite Nockenfläche einen zweiten Schalter 11 im Schalterstück 9. Vorteilhafterweise sind die Nocken 18 einer Nockenfläche 16, 17 so gestaltet, dass während der Drehbewegung des Bedienknopfes 2 die in der Ebene eines Nockens 18 liegende Schalter 10, 11 jeweils über einen ersten Drehwinkel ein- und über einen zweiten Drehwinkel ausgeschaltet sind. Die durch die Nocken 18 verursachte Umschaltung der Schalter 10, 11 erfolgt jeweils kurz vor dem Ende der Teildrehung. Mit den Nocken 18 wird, wie später gezeigt wird, jeweils die nächste Injektionsdosis eingestellt und angezeigt.

Andere Schaltmittel sind als Schaltringe 8 ausgebildet. Diese machen die Drehbewegung des Bedienknopfes 2 nicht mit. Das wird beispielsweise dadurch verhindert, dass ein Ansatz 20 des Schaltringes 8 fest in einem Loch der Gehäusewand 5 sitzt. Auch durch den Schaltring 8 geht der zylindrische Teil des Bedienknopfes 2 hindurch.

Ein Schaltring 8 hält in der Ruhelage und während des Übergangs von der Ruhelage zur Endlage und zurück bei der axialen Bewegung des Bedienknopfes 2 einen dritten Schalter 12 des Schalterstückes 9 in einer ersten Stellung. In der nicht gezeichneten Endstellung der axialen Bewegung des Bedienknopfes 2 bringt er den dritten Schalter 12 des Schalterstückes 9 in eine zweite Stellung. Dazu trägt der zylindrische Teil 3 des Bedienknopfes 2 beispielsweise einen Ringnut 19, in den der Schaltring 8 in der Endlage der axialen Bewegung eingreift und dabei den dritten Schalter 12 in die zweite Stellung bringt.

Dies wird im dargestellten Beispiel dadurch bewirkt, dass der Schaltring 8 durch eine Feder hinter dem Schalter 12 in die Ringnut 19 gedrückt wird und Platz freigibt, so dass gleichzeitig der unter dem Druck der Feder liegende Schalter 12 von der ersten Stellung in die zweite Stellung umschaltet. Da die Endstellung der axialen Bewegung jeweils nur am Ende einer Injektion eingenommen wird, sind durch den Schaltring 8 die Anzahl der abgegebenen Injektionen zählbar.

Die Nockenringe 7 und die Schaltringe 8 wirken über die Schalter 10, 11, 12 auf einen elektrischen Teil der Anzeigeeinrichtung, die im wesentlichen aus einer Leiterplatte 13, einer Batterie 14 und einer LCD-Anzeigeeinrichtung 15 besteht. Die Leiterplatte 13 enthält einen Prozessor, auf den die Schalterstellungen der Schalter 10, 11, 12 einwirken und mit dem je
- eine Anzeigedauer-Zählung,
- eine Zählung der Teildrehungen beim Einstellen einer Injektionsdosis,
- eine Zählung einer "Reserve", berechnet aus der Differenz zwischen dem Volumen der beim Einsetzen der Ampulle vorhanden gewesenen Injektionsflüssigkeit minus den bis zum gegebenen Zeitpunkt insgesamt eingestellten Injektionsdosen auf der Basis der Teildrehungen und
- eine Zählung der ausgegebenen Injektionsdosen auf der Basis der Teildrehungen
   durchführbar ist.
   Durch die Anzeigeeinrichtung wird so die Zahl der Teildrehungen des Bedienknopfes (2) zum Vergrössern der einstellbaren Injektionsdosis über den gesamten Inhaltsbereich des Flüssigkeitsbehälters oder der mechanisch vorgegebenen Dosis addiert, bei Umkehr der Drehrichtung subtrahiert.

Die Anzeigedauer-Zählung bestimmt die Dauer, während der eine der oben aufgeführten Grössen an der LCD-Anzeigeeinrichtung (15) anzeigbar ist, falls nicht vorher eine andere Grösse dort zur Anzeige gelangt. Beispielsweise beträgt diese Dauer bei dem dargestellten Gerät 128 Sekunden.

In dem als Beispiel gezeigten Gerät 1 nach der Fig. 1 und 2 werden beim Einstellen der Injektionsdosis die Teildrehungen des Bedienknopfes 2, es sind, wie weiter oben bereits gesagt, Viertelsdrehungen, über einen Nockenring 7 und Schalter 10, 11 mit einem 2-stelligen Dualzähler des Prozessors gezählt und, dabei entsprechend der Stellung des Bedienknopfes 2 die Dualzahlen "00", "10", "11" und "01" an der LCD-Anzeigeinrichtung 15 ausgegeben.

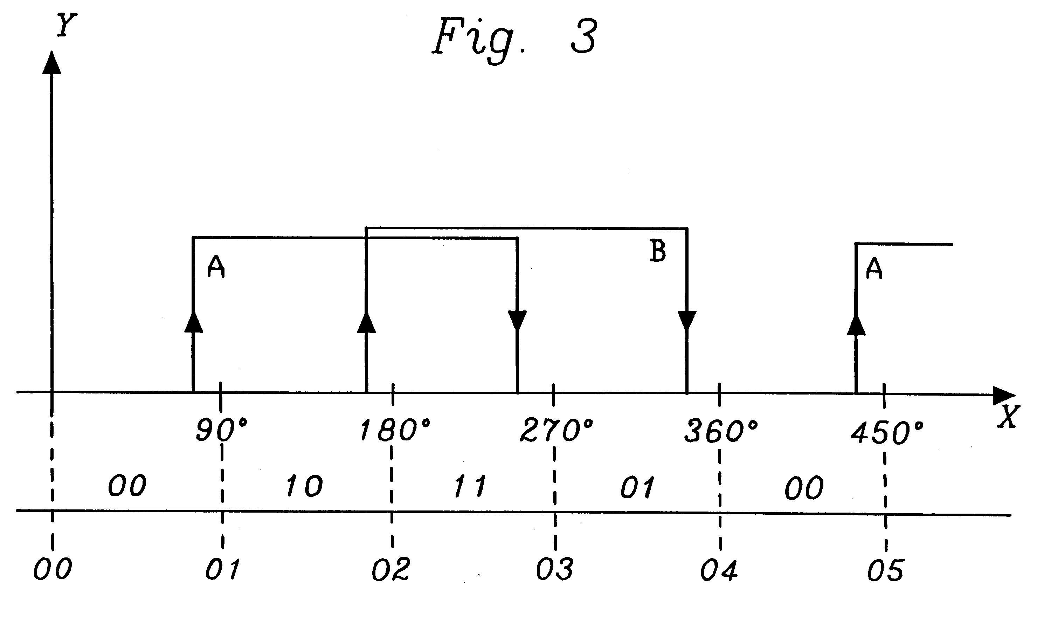

Da, wie ebenfalls weiter oben gesagt wurde, die durch die Drehung des Bedienknopfs 2, den Nockenring 7 und die Nocken 18 betätigten ersten und zweiten Schalter 10, 11 kurz vor dem Ende der entsprechenden Teildrehungen umgeschaltet werden, kann der Bedienknopf 2 beliebig im Uhrzeigersinn und im Gegenunrzeigersinn gedreht werden, ohne dass Fehler im Erkennen seiner Stellung durch Ablesen der LCD-Anzeigeeinrichtung entstehen. Die Art der Signalbildung wird in der Fig. 3 gezeigt.

Dort ist auf einer X-Achse der Drehwinkel, auf der Y-Achse die Signalgrössen am Anzeigeausgang für die Einstellung der Injektionsdosis angegeben, in der Zeile darunter wird die zugehörige Anzeige eines Dualzählers, darunter die zugehörige Anzeige eines LCD-Zählers angezeigt.

Kurz vor dem Ende der ersten Teildrehung (vor 90°) schaltet der erste Nocken 18 der ersten Nockenfläche 16 den ersten Schalter 10 und setzt das erste Signal A hoch. Es erscheint beispielsweise an der in diesem Falle dual zählenden LCD-Anzeigeeinrichtung 15 die Dualzahl "10".

Kurz vor dem Ende der zweiten Teildrehung (vor 180°) schaltet der zweite Nocken 18' der zweiten Nockenfläche 17 den zweiten Schalter 11 und setzt das zweite Signal B hoch. Das erste Signal bleibt immer noch hoch. Es erscheint an der LCD-Anzeigeeinrichtung 15 die Dualzahl "11".

Kurz vor dem Ende der dritten Teildrehung (vor 270°) schaltet der erste Nocken 18 der ersten Nockenfläche 16 den ersten Schalter 10 zurück und setzt das erste Signal A tief. Das zweite Signal bleibt immer noch hoch. Es erscheint an der LCD-Anzeigeeinrichtung 15 die Dualzahl "01".

Kurz vor dem Ende der vierten Teildrehung (vor 360°) schaltet der zweite Nocken 18' der zweiten Nockenfläche 17 den zweiten Schalter 11 zurück und setzt das Zweite Signal B tief. Das erste Signal bleibt tief. Es erscheint an der LCD-Anzeigeeinrichtung 15 die Dualzahl "00".

Dies läuft so weiter, bis die Drehbewegung des Bedienknopfes 2 nach Erreichen der einzustellenden Injektionsdosis beendet wird. Die Dualzahlen können auch in einer anderen Reihenfolge angegeben werden.

Die Zahl der Teilumdrehungen wird ferner mit einem LCD-Zähler aufsummiert und erscheint als Dezimalzahl nach Ende der Drehbewegung an der LCD-Anzeigeeinrichtung 15. Sie gibt die Grösse der eingestellten Injektionsdosis, basierend auf die Zahl der Teilumdrehungen, an. Diese Anzeige wird in der Endstellung der axialen Bewegung des Bedienknopfes 2, also nach der Injektion der eingestellten Injektionsdosis, über den Schaltring 8 und den dritten Schalter 12 vom Prozessor mit der Zeitverzögerung der Anzeigedauer-Zählung gelöscht.

Die Zahl der injizierten Teilumdrehungen wird von einem Reserve-Zähler abgezogen, der bei dem Einsetzen einer neuen Ampulle jeweils auf die gesamte mit dieser Ampulle ausgebbare Injektionsmenge, basierend auf der Zahl der möglichen Teilumdrehungen, selbsttätig eingestellt wird. Dieser zeigt ein auffallendes Sonderzeichen, beispielsweise ein flackerndes "tt" statt der eingestellten Dosis an der LCD-Anzeigeeinrichtung 15, wenn die eingestellte Dosis grösser als die noch vorhandene Reserve der Injektionsflüssigkeit ist.

Der Prozessor kann einen Timer enthalten. Ist die maximal zulässige Zeit für die Abgabe einer Injektion aus der eingesetzten Ampulle überschritten, so ist an der LCD-Anzeigeeinrichtung 15 ein auffallendes Sonderzeichen, beispielsweise ein flackerndes "bb" anzeigbar, nachdem versucht wurde, eine Injektionsdosis einzustellen.

Weiter überwacht der Prozessor die Leistungsabgabe der Batterie. Ist die Leistung zu klein geworden, so zeigt er ein auffallendes Sonderzeichen, beispielsweise ein flackerndes "bt", an der LCD-Anzeigeeinrichtung (15), nachdem versucht wurde, eine Injektionsdosis einzustellen.

Statt eines Prozessors auf der Leiterplatte 13 können auch diskrete Schaltungen einer vorgegebenen Organisation auf der Leiterplatte 13 die genannten Funktionen ausüben. Es kann daher in allgemeinerer Form davon gesprochen werden, dass die genannten Funktionen von der Leiterplatte 13 durchführbar sind.

Es sind noch andere vorteilhafte Anzeigemöglichkeiten mit der Einrichtung durchführbar. Es ist weiterhin möglich, die Anzeigeeinrichtung in ein Injektionsgerät einzubauen, bei dem mit elektrischen Mitteln die Injektionsdosis einstellbar und die Injektion ausführbar ist.

Mit einem Injektionsgerät mit der erfindungsgemässen Anzeigeeinrichtung kann der Patient nach Einstellen der gewünschten Dosis diese nachträglich fehlerfrei überprüfen und er erhält eine Information, wie viele Injektionsflüssigkeit noch für weitere Injektionen zur Verfügung steht. Sie erfüllt somit die gestellte Aufgabe.

## Patentansprüche

1. Anzeigeeinrichtung an einem Injektionsgerät (1) zum Injizieren jeweils wählbarer Injektionsdosen aus einem mit einem Kolben ausgerüsteten Flüssigkeitsbehälter, insbesondere einer Ampulle, das einen axialen, einen zylindrischen Teil (3) besitzenden Bedienknopf (2) aufweist, an dem Drehbewegungen zur Einstellung der Grösse einer anschliessend zu injizierenden Injektionsdosis und darauf axiale Bewegungen zum Durchführen der Injektion ausführbar sind, wobei sich die Drehbewegungen des Bedienknopfs (2) aus Teildrehungen zusammensetzt, von denen eine gegebene Zahl eine ganze Umdrehung ergibt, dadurch gekennzeichnet, dass
A) mit dem Bedienknopf (2) durch dessen Bewegung betätigbare Schaltmittel (7, 8) verbunden sind, die in ein Schalterstück (9) eingreifen und dort Schalter (10, 11, 12) betätigen, die Schalter (10, 11, 12) des Schalterstücks (9) elektrisch mit einer Leiterplatte (13) verbunden sind die durch eine Batterie (14) versorgt ist, und die Leiterplatte (13) mit einer LCD-Anzeigeeinrichtung (15) verbunden ist,
B) durch die Anzeigeeinrichtung die Zahl der Teildrehungen des Bedienknopfes (2) zum Vergrössern der einstellbaren Injektionsdosis über den gesamten Inhaltsbereich des Flüssigkeitsbehälters oder der mechanisch vorgegebenen Dosis addiert, bei Umkehr der Drehrichtung subtrahiert werden, und
C) eine axiale Bewegung des Bedienknopfes (2) nur am Ende einer solchen Teildrehung ausführbar ist.

2. Einrichtung nach Anspruch 1, dadurch gekennzeichnet, dass der zylindrische Teil (3) des Bedienknopfes (2) eine der Anzahl der Teildrehungen für eine ganze Umdrehung entsprechende Zahl von in axialer Richtung verlaufenden Längsnocken (4) trägt, die bei seiner axialen Bewegung in ebenso vielen Längsnuten (6) einer fest angebrachten Gehäusewand (5) eingreifen, während Drehbewegungen nur ausführbar sind, wenn kein Eingriff zwischen den Längsnocken (4) und den Längsnuten (6) stattfindet.

3. Einrichtung nach Anspruch 1, dadurch gekennzeichnet, dass Schaltmittel als Nockenringe (7) ausgebildet sind, welche die Drehbewegungen des Bedienknopfes (2) mitmachen und durch die der zylindrische Teil (3) des Bedienknopfes (2) hindurchgeht.

4. Einrichtung nach Anspruch 3, dadurch gekennzeichnet, dass die Nockenringe (7) während der Drebbewegung des Bedienknopfes (2) durch die Längsnocken (4) am Bedienknopf (2) mitgenommen werden.

5. Einrichtung nach Ansprüche 3, dadurch gekennzeichnet, dass ein Nockenring (7) auf seinem äusseren Umfang eine oder mehrere, dann in Umfangsrichtung nebeneinander liegende, Nockenflächen (16, 17) mit Nocken (18) trägt, wobei die Nocken (18) der einen Nockenfläche (16) versetzt zu den Nocken (18) der anderen Nockenflächen (17) liegen und die Nocken (18) einer Nockenfläche (16, 17) jeweils nur einen ersten oder einen zweiten Schalter (10, 11) des Schalterstücks (9) betätigen.

6. Einrichtung nach Anspruch 5, dadurch gekennzeichnet, dass die Nockenringe (7) so gestaltet sind, dass während der Drehbewegung des Bedienknopfes (2) die in einer Nockenfläche (16, 17) liegenden Schalter (10, 11) im schalterstück (9) jeweils über einen ersten Drehwinkel ein- und über einen zweiten Drehwinkel ausschaltbar sind.

7. Einrichtung nach Anspruch 6, dadurch gekennzeichnet, dass die durch die Nocken (18) verursachte Umschaltung der ersten oder zweiten Schalter (10, 11) jeweils kurz vor dem Ende einer Teildrehung erfolgt.

8. Einrichtung nach Anspruch 1, dadurch gekennzeichnet, dass Schaltmittel als Schaltringe (8) ausgebildet sind, welche weder die Drehbewegung noch die axiale Bewegung des Bedienknopfes (2) mitmachen und durch die der zylindrische Teil des Bedienknopfes (2) hindurchgeht.

9. Einrichtung nach Anspruch 8, dadurch gekennzeichnet, dass der Schaltring (8) in einer Ruhestellung der axialen Bewegung des Bedienknopfes (2) und wahrend des Übergangs von der Ruhestellung zu einer Endstellung und umgekehrt einen dritten Schalter (12) des Schalterstückes (9) in der einen Schaltstellung hält.

10. Einrichtung nach Anspruch 9, dadurch gekennzeichnet, dass der Schaltring (8) in der Endstellung der axialen Bewegung des Bedienknopfes (2) den dritten Schalter (12) des Schalterstückes (9) in die andere Schaltstellung bringt.

11. Einrichtung nach Anspruch 10, dadurch gekennzeichnet, dass der zylindrische Teil (3) des Bedienknopfes (2) eine Ringnut (19) trägt, in den der Schaltring (8) in der Endstellung der axialen Bewegung eingreift und dabei den dritten Schalter (12) in die andere Schaltstellung bringt.

12. Einrichtung nach Anspruch 1, dadurch gekennzeichnet, dass die Leiterplatte (13) einen Prozessor oder entsprechende diskrete Elemente enthält, in denen die Schaltstellungen der Schalter (10, 11, 12) eingebbar sind und mit dem je eine Anzeigedauer-Zählung, eine Zählung der Teildrehungen beim Einstellen einer Injektionsdosis, eine Zählung einer Reserve, berechnet aus der Differenz zwischen dem Volumen der beim Einsetzen der Ampulle vorhanden gewesenen Injektionsflüssigkeit minus den bis zum gegebenen Zeitpunkt insgesamt eingestellten Injektionsdosen auf der Basis der Teildrehungen und eine Zählung der ausgegebenen Injektionsdosen auf der Basis der Teildrehungen durchführbar ist.

13. Einrichtung nach Anspruch 12, dadurch gekennzeichnet, dass die mit der Leiterplatte (13) elektrisch verbundene LCD-Anzeigeeinrichtung (15) die Zahl der Teildrehungen beim Einstellen der Injektionsdosis anzeigt.

14. Einrichtung nach Anspruch 12, dadurch gekennzeichnet, dass die Leiterplatte (13) die Zahl der Teildrehungen, die der Summe der ausgegebenen Injektionsdosen entspricht, bildet und die LCD-Anzeigeeinrichtung (15) diese anzeigt.

15. Einrichtung nach Anspruch 12, dadurch gekennzeichnet, dass die Leiterplatte (13) die Zahl der Teildrehungen, die der Reserve der Injektionsflüssigkeit entspricht, bildet und die LCD-Anzeigeeinrichtung (15) diese anzeigt.

16. Einrichtung nach Anspruch 15, dadurch gekennzeichnet, dass durch den Prozessor ein auffälliges erstes Sonderzeichen abgegeben wird, wenn die Reserve kleiner ist als die eingestellte Injektionsdosis.

17. Einrichtung nach Anspruch 12, dadurch gekennzeichnet, dass durch den Prozessor dann ein auffälliges zweites Sonderzeichen abgegeben wird, wenn die längste zulässige Zeit seit dem Einsetzen der Ampulle abgelaufen ist.

18. Einrichtung nach Anspruch 12, dadurch gekennzeichnet, dass durch den Prozessor die Batterie (14) überprüfbar ist und ein auffälliges drittes Sonderzeichen abgegeben wird, wenn ihre Leistung unter ein zulässiges Mass abgesunken ist.

19. Einrichtung nach einem der Ansprüche 12 bis 18, dadurch gekennzeichnet, dass die Anzeigedauer-Zählung nach einer gegebenen Zeitdauer nach dem Erscheinen einer Anzeige an der LCD-Anzeigeeinrichtung diese löscht, falls nicht in der Zwischenzeit eine andere Grösse angezeigt ist, und die Anzeigeeinrichtung mit kleinerem Energieverbrauch weiterläuft.

20. Einrichtung nach Anspruch 1 oder 12, dadurch gekennzeichnet, dass auf der Leiterplatte (13) diskrete Schaltungen einer vorgegebenen Organisation vorgesehen sind, welche die Funktion eines Prozessors ausüben.

## Claims

1. A display for an injection device (1) used to inject particular, selectable doses from a plunger-fitted, liquid-receptacle, especially an ampoule, said device comprising an operating head (2) with an axial, cylindrical part (3), said head being rotatable to set the magnitude of the injection dose and thereupon to be axially displaced to carry out the injection, the rotation of this operating head (2) being composed of fractional turns of which a given number amounts to one revolution, characterized in that
(A) switch means (7, 8) are connected to the operating head (2) of which the motion actuates them, said switch means (7, 8) engaging a switch part (9) and there actuate switches (10, 11, 12), the switches (10, 11, 12) of the switch part (9) being connected electrically with a printed-circuit board (13) powered by a battery (14) and the printed-circuit board (13) being connected to an LCD display system (15),
(B) by means of the display, the number of fractional turns of the operating head (2) is added over the entire capacity range of the liquid receptacle or of the mechanically preset dose to increase the adjustable injection dose, or, is subtracted if the direction of rotation is reversed,
(C) the operating head (2) can be axially displaced only at the end of such a fractional turn.

2. Display according to claim 1, characterized in that the cylindrical part (3) of the operating head (2) comprises such a number of axial, lengthwise bosses (4) that it corresponds to the number of fractional turns required for one revolution, said bosses (4) engaging in just as many lengthwise channels (6) in a stationary housing wall (5) during the axial displacement of said operating head (2), whereas rotation is possible only in the absence of engagement between the lengthwise bosses (4) and the lengthwise channels (6).

3. Display according to claim 1, characterized in that switch means are present in the form of cam rings (7) which participate in the rotation of the operating head (2) and which are crossed by the cylindrical part (3) of the operating head (2).

4. Display according to claim 3, characterized in that the lengthwise bosses (4) at the operating head (2) drive the cam rings (7) into joint rotation during the rotation of the operating head (2).

5. Display according to claim 3, characterized in that one cam ring (7) evinces at its outside circumference one or more circumferentially adjoining cam surfaces (16, 17) with bosses (18), the bosses (18) of one cam surface (16) being offset relative to the bosses (18) of the other cam surface (17) and the bosses (18) of one cam surface (16, 17) each time actuating one first or one second switch (10, 11) of the switch part (9).

6. Display according to claim 5, characterized in that the cam rings (7) are designed in such manner that during the rotation of the operating head (2), the switches (10, 11) in the switch part (9) located in the cam surface (16, 17) can be turned ON by means of a first angular excursion and turned OFF by means of a second angular excursion.

7. Display according to claim 6, characterized in that the switching reversal implemented by the bosses (18) of the first or second switch (10, 11) occurs each time shortly before the end of fractional turn.

8. Display according to claim 1, characterized in that switch means are designed as switch rings (8) which participate neither in the rotation nor in the axial displacement of the operating head (2) and which are crossed by the cylindrical part of the operating head (2).

9. Display according to claim 8, characterized in that for an axial rest position of the operating head (2), and during the transit from said rest position to an axial end position and vice-versa, the switch ring (8) holds a third switch (12) of the switch part (9) into the other switch position.

10. Display according to claim 9, characterized in that the switch ring (8) when in the axial end position of the operating head (2) moves the third switch (12) of the switch part (9) into the other switch position.

11. Display according to claim 10, characterized in that the cylindrical part (3) of the operating head (2) comprises an annular channel (19) entered by the switch ring (8) for the axial end position of said operating head, said switch ring (8) thereby moving the third switch (2) into the other switch position.

12. Display according to claim 1, characterized in that the printed-circuit board (13) comprises a processor or corresponding discrete components receiving as inputs the switch positions of the switches (10, 11, 12) and allowing counting the duration of display, counting fractional turns when setting the injection dose, counting a reserve based on the difference between the volume of injection liquid present at ampoule insertion and the total of the injection doses set up the particular time based on the fractional turns and counting the outputs of injection doses based on the fractional turns.

13. Display according to claim 12, characterized in that the LCD display system (15) electrically connected to the printed-circuit board (13) displays the number of fractional turns when setting the injection dose.

14. Display according to claim 12, characterized in that the printed-circuit board (13) forms the number of fractional turns corresponding to the sum of issued injection doses and that the LCD display system (15) displays said number.

15. Display according to claim 12, characterized in that the printed-circuit board (13) forms the number of fractional turns corresponding to the injection-liquid reserve and that the LCD display system (15) displays said number.

16. Display according to claim 15, characterized in that a first special alarm character is emitted by the processor when the reserve is less than the set injection dose.

17. Display according to claim 16, characterized in that a second special alarm character is emitted by the processor when the maximum admissible time from ampoule insertion has lapsed.

18. Display according to claim 17, characterized in that the processor is able to check the battery (14) and will emit a third special alarm character if the battery output has dropped below an admissible level.

19. Display according to one of the claims 12 through 18, characterized in that the display duration count will erase a display at the LCD display system (15) a certain time after it has appeared there unless meantime another value was displayed and the display continues at lesser power drain.

20. Display according to claim 1 or 12, characterized in that discrete circuits of predetermined functions are present on the printed-circuit board (13) and serve as a processor.

## Revendications

1. Dispositif d'affichage sur un appareil d'injection (1) pour l'injection de doses d'injection chaque fois sélectionnables provenant d'un récipient à liquide équipé d'un piston, en particulier une ampoule, qui présente une tête de service (2) axiale qui possède une partie cylindrique (3), et sur laquelle peuvent être effectués des déplacements en rotation pour le réglage de la valeur d'une dose d'injection qui doit ensuite être injectée, et ensuite des déplacements axiaux pour la réalisation de l'injection, les déplacements en rotation de la tête de service (2) étant constitués de la combinaison de rotations partielles à partir d'un nombre défini desquelles s'obtient un tour complet,
caractérisé en ce que:
A) à la tête de service (2) sont reliés des moyens de commutation (7, 8) qui peuvent être activés par le déplacement de celle-ci, qui s'engagent dans une pièce à commutateurs (9) et y actionnent des commutateurs (10, 11, 12), les commutateurs (10, 11, 12) de la pièce à commutateurs (9) étant reliés électriquement à une plaque conductrice (13) qui est alimentée par une batterie (14), et la plaque conductrice (13) est reliée à un dispositif d'affichage (15) à cristaux liquides,
B) le nombre des rotations partielles de la tête de service (2) est additionné par le dispositif d'affichage pour augmenter la dose d'injection réglable sur toute la plage de contenance du récipient à liquide ou de la dose prédéterminée mécaniquement, ou est soustrait en cas d'inversion du sens de rotation, et
C) un déplacement axial de la tête de service (2) ne peut être effectué qu'à la fin d'une telle rotation partielle.

2. Dispositif selon la revendication 1, caractérisé en ce que la partie cylindrique (3) de la tête de service (2) porte un nombre, qui correspond au nombre de rotations partielles d'un tour complet, de nervures longitudinales (4) qui s'étendent dans le sens axial et qui, lors d'un déplacement axial de la tête de service (2), s'engagent dans des rainures longitudinales (6), en nombre identique, d'une paroi de boîtier (5) installée fixement, tandis que des déplacements en rotation ne peuvent être effectués que lorsqu'il n'y a pas d'engagement entre les nervures longitudinales (4) et les rainures longitudinales (6).

3. Dispositif selon la revendication 1, caractérisé en ce que les moyens de commutation sont configurés comme bagues à cames (7) qui accompagnent les déplacements en rotation de la tête de service (2) et qui sont traversées par la partie cylindrique (3) de la tête de service (2).

4. Dispositif selon la revendication 3, caractérisé en ce que pendant le déplacement en rotation de la tête de service (2), les bagues à cames (7) sont entraînées par les nervures longitudinales (4) de la tête de service (2).

5. Dispositif selon la revendication 3, caractérisé en ce qu'une bague à cames (7) présente sur sa périphérie externe une ou plusieurs surfaces de came (16, 17) présentant une came (18), ces surfaces de came (18) étant disposées l'une à côté de l'autre quand il en existe plusieurs, les cames (18) d'une surface de came (16) étant décalées par rapport aux cames (18) de l'autre surface de came (17), et les cames (18) d'une surface de came (16, 17) n'actionnant chaque fois qu'un premier ou un second commutateur (10, 11) de la pièce à commutateurs (9).

6. Dispositif selon la revendication 5, caractérisé en ce que les bagues à cames (7) sont configurées de telle sorte que pendant le déplacement en rotation de la tête de service (2), les commutateurs (10, 11) de la pièce à commutateurs (9) situés dans une surface de came (16, 17) peuvent être chaque fois branchés sur un premier angle de rotation et débranchés sur un second angle de rotation.

7. Dispositif selon la revendication 6, caractérisé en ce que la commutation du premier ou du second commutateur (10, 11) provoquée par les cames (18) s'effectue chaque fois un peu avant la fin d'une rotation partielle.

8. Dispositif selon la revendication 1, caractérisé en ce que les moyens de commutation sont configurés comme bagues à cames (8) qui n'accompagnent ni le déplacement en rotation ni le déplacement axial de la tête de service (2) et qui sont traversées par la partie cylindrique de la tête de service (2).

9. Dispositif selon la revendication 8, caractérisé en ce que la bague à cames (8) maintient un troisième commutateur (12) de la pièce à commutateurs (9) respectivement dans une position de commutation et dans l'autre lorsque le déplacement axial de la tête de service (2) est en position de repos et pendant le passage de la position de repos à une position d'extrémité.

10. Dispositif selon la revendication 9, caractérisé en ce que la bague à cames (8) amène le troisième commutateur (12) de la pièce à commutateurs (9) dans l'autre position de commutation lorsque le déplacement axial de la tête de service (2) atteint la position d'extrémité.

11. Dispositif selon la revendication 10, caractérisé en ce que la partie cylindrique (3) de la tête de service (2) porte une rainure annulaire (19) dans laquelle la bague à cames (8) s'engage dans la position d'extrémité du déplacement axial, et amène alors le troisième commutateur (12) dans l'autre position de commutation.

12. Dispositif selon la revendication 1, caractérisé en ce que la plaque conductrice (13) contient un processeur ou des éléments discrets correspondants dans lequel ou dans lesquels les positions de commutation des commutateurs (10, 11, 12) peuvent être introduites, et par lequel ou lesquels il est possible d'effectuer un comptage de la durée d'affichage, un comptage des rotations partielles lors du réglage d'une dose d'injection, un comptage d'une réserve, calculée à partir de la différence entre le volume de liquide d'injection existant lors de l'insertion de l'ampoule et la somme, basée sur les rotations partielles, des doses d'injection établies jusqu'à l'instant donné, ainsi qu'un comptage, basé sur les rotations partielles, des doses d'injection émises.

13. Dispositif selon la revendication 12, caractérisé en ce que le dispositif d'affichage (15) à cristaux liquides relié électriquement à la plaque conductrice (13) affiche le nombre des rotations partielles lors du réglage de la dose d'injection.

14. Dispositif selon la revendication 12, caractérisé en ce que la plaque conductrice (13) forme le nombre des rotations partielles qui correspond à la somme des doses d'injection émises, et le dispositif d'affichage (15) à cristaux liquides affiche cette somme.

15. Dispositif selon la revendication 12, caractérisé en ce que la plaque conductrice (13) forme le nombre des rotations partielles qui correspond à la réserve en liquide d'injection, et le dispositif d'affichage (15) à cristaux liquides affiche cette réserve.

16. Dispositif selon la revendication 15, caractérisé en ce qu'un premier caractère spécial d'avertissement est émis par le processeur lorsque la réserve est plus petite que la dose d'injection réglée.

17. Dispositif selon la revendication 12, caractérisé en ce qu'un second caractère spécial d'avertissement est émis par le processeur lorsque la plus grande durée admissible depuis l'insertion de l'ampoule est écoulée.

18. Dispositif selon la revendication 12, caractérisé en ce que la batterie (14) peut être surveillée par le processeur, et un troisième caractère spécial d'avertissement est émis par le processeur lorsque sa puissance est descendue en-deçà d'une valeur admissible.

19. Dispositif selon l'une quelconque des revendications 12 à 18, caractérisé en ce que le comptage de la durée d'affichage efface l'affichage après écoulement d'une durée donnée depuis l'apparition d'un affichage sur le dispositif d'affichage à cristaux liquides au cas où aucune autre valeur n'a été affichée entre-temps, et le dispositif d'affichage continue à fonctionner avec une consommation d'énergie réduite.

20. Dispositif selon la revendication 1 ou 12, caractérisé en ce que sur la plaque conductrice (13) sont prévus des circuits distincts d'un système qui joue le rôle d'un processeur.
